(19)

Europäisches
Patentamt

European
Patent Office

Office européen
des brevets

(11)  EP 2 861 614 B1

(12)  EUROPEAN PATENT SPECIFICATION

(45) Date of publication and mention
of the grant of the patent:
**30.11.2016  Bulletin 2016/48**

(21) Application number: **13732317.6**

(22) Date of filing: **12.06.2013**

(51) Int Cl.:
***C07K 1/22*** *(2006.01)*  ***C12N 9/64*** *(2006.01)*

(86) International application number:
**PCT/US2013/045496**

(87) International publication number:
**WO 2013/188587 (19.12.2013 Gazette 2013/51)**

(54) **METHOD FOR PURIFICATION OF RECOMBINANT FACTOR Xa DERIVATIVES**

VERFAHREN ZUR AUFREINIGUNG REKOMBINANTER FAKTOR-Xa-DERIVATE

PROCÉDÉ DE PURIFICATION DE DÉRIVÉS DU FACTEUR Xa RECOMBINANTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.06.2012  US 201261659821 P**

(43) Date of publication of application:
**22.04.2015  Bulletin 2015/17**

(73) Proprietor: **Portola Pharmaceuticals, Inc.
South San Francisco, California 94080 (US)**

(72) Inventors:
• **LU, Genmin
Burlingame, California 94010 (US)**
• **SINHA, Uma
San Francisco, California 94127 (US)**

(74) Representative: **Srinivasan, Ravi Chandran
J A Kemp
14 South Square
Gray's Inn
London WC1R 5JJ (GB)**

(56) References cited:
**US-A1- 2010 255 000**

• **BOCK P E ET AL: "Isolation of human blood
coagulation alpha-factor Xa by soybean trypsin
inhibitor-Sepharose chromatography and its
active-site titration with fluorescein
mono-p-guanidinobenzoate", ARCHIVES OF
BIOCHEMISTRY AND BIOPHYSICS, ACADEMIC
PRESS, US, vol. 273, no. 2, 1 September 1989
(1989-09-01), pages 375-388, XP024815534, ISSN:
0003-9861, DOI: 10.1016/0003-9861(89)90496-7
[retrieved on 1989-09-01]**

**Description**

**BACKGROUND**

Anticoagulants serve a need in the marketplace in treatment or prevention of undesired thrombosis in patients with a tendency to form blood clots, such as, for example, those patients having clotting disorders, confined to periods of immobility or undergoing medical surgeries. One of the major limitations of anticoagulant therapy, however, is the bleeding risk associated with treatment, and limitations on the ability to rapidly reverse the anticoagulant activity in case of overdosing or if an urgent surgical procedure is required. Thus, specific and effective antidotes to all forms of anticoagulant therapy are highly desirable. For safety considerations, it is also advantageous to have an anticoagulant-antidote pair in the development of new anticoagulant drugs.

Previously reported modified derivatives of factor Xa (fXa) proteins are useful as antidotes to anticoagulants targeting fXa, such as those described in US Patent No. 8,153,390 and 8,268,783. The modified derivatives of fXa proteins bind to and/or substantially neutralize the anticoagulant. Certain modifications introduced to these fXa derivatives, however, pose several challenges for purification since conventional methods for purification of clotting factors may not be effective for these modified fXa proteins.

**SUMMARY**

Disclosed herein are methods and kits for purifying a serine protease. The methods, in some embodiments, entail loading the serine protease to a soybean trypsin inhibitor (STI)-based affinity chromatograph, such as a resin or a column, and eluting the polypeptide with an elution buffer. The elution buffer comprises a competitive agent, that disrupts interaction between the STI and the serine protease which is arginine. In some embodiments, the elution buffer further comprises a salt and/or a detergent.

In one embodiment, provided is a method of purifying a serine protease comprising loading the serine protease to a soybean trypsin inhibitor (STI)-based affinity chromatograph, and eluting the serine protease with an elution buffer comprising serine that disrupts interaction between the STI and the serine protease.

In some aspects, the elution buffer further comprises a salt, a detergent and/or a chaotropic agent.

In some aspects of the disclosure, the agent is a competitive agent that can be selected from the group consisting of benzamidine, p-aminobenzamidine, arginine, a small molecule fXa inhibitor, a peptide fXa inhibitor, and a peptidomimetic fXa inhibitor. The competitive agent is arginine.

In some aspects, the serine protease is a polypeptide comprising the amino acid sequence of SEQ ID NO: 1 or 2, or a polypeptide having at least about 80% sequence identity to SEQ ID NO: 1 or 2, having a deletion of at least part of the Gla domain and a mutation at the active site.

In some aspects, the serine protease comprises the amino acid sequence of SEQ ID NO: 2. or a polypeptide having at least about 95% sequence identity to SEQ ID NO: 2, having a deletion of at least part of the Gla domain and a mutation at the active site. In some aspects, the serine protease comprises the amino acid sequence of SEQ ID NO: 2.

In some aspects, the pH of the elution buffer is from about 4.5 to about 10.5. In some aspects, the pH of the elution buffer is about 5.0. In some aspects, the pH of the elution buffer is about 7.4.

In some aspects, the elution buffer comprises from about 250 mM arginine to about 1000 mM arginine. In some aspects, the elution buffer comprises about 500 mM arginine. In some aspects, the pH of the elution buffer is about 5.0.

In some aspects, the method further comprises subjecting the eluted serine protease to purification with an ion-exchange column.

Purified serine proteases are also disclosed that are prepared by the method of the present disclosure.

In one embodiment, provided is a kit comprising a soybean trypsin inhibitor (STI)-based affinity chromatograph, and an elution buffer comprising arginine that disrupts the interaction between the STI and a serine protease.

In some aspects, the pH of the elution buffer is from about 4.5 to about 10.5. In some aspects, the elution buffer comprises from about 250 mM arginine to about 1000 mM arginine. In some aspects, the kit further comprises a wash buffer comprising about 250 mM NaCl at a neutral pH.

**BRIEF DESCRIPTION OF THE FIGURES**

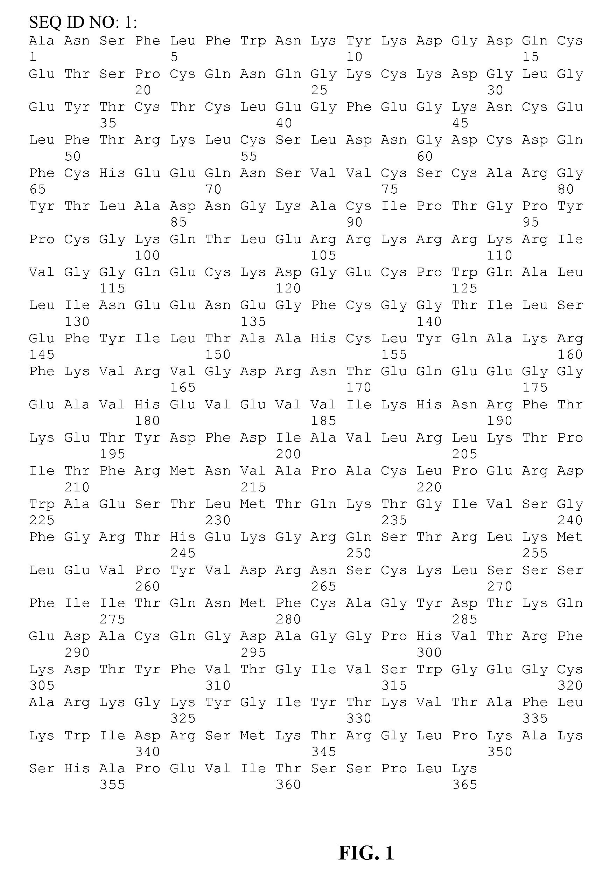

**FIG. 1** shows SEQ ID NO: 1, a fXa derivative (also referred to as r-Antidote precursor) with the linker, -RKRRKR- (SEQ ID NO: 3) at amino acids 106-111.

**FIG. 2** shows SEQ ID NO: 2, a fXa derivative (also referred to as r-Antidote) with the linker removed from the r-Antidote precursor.

**FIG. 3** shows the loading profile using purified r-Antidote on a Benzamidine-Agarose affinity resin as described in Example 1.

**FIG. 4** shows the loading profile using purified r-Antidote on an L-Lysine-Agarose affinity resin as described in Example 1.

**FIG. 5** shows the loading profile using purified r-Antidote on an Aprotinin-Agarose affinity resin as described in Example 1.

**FIG. 6** shows the loading profile using purified r-Antidote on a STI-Agarose affinity resin as described in Example 1.

**FIG. 7** shows the STI-Agarose loading profile using conditioned media harvested from cell culture (harvested cell culture fluid), demonstrating that the functional protein is captured by the STI resin (as described in Example 2).

**FIG. 8** shows the loading profile using harvested cell culture fluid on a HQ-Sepharose pre-column (in flow-through mode) as described in Example 2.

**FIG. 9** shows the loading profile of HQ-sepharose FT-fractions loaded to STI-Agarose demonstrating that the functional protein is captured by STI-resin.

**FIG. 10A-B** show that the bound proteins are eluted with 1M benzamidine as described in Example 2. The elution profile using 1 M benzamidine is shown in FIG. 10A, and a Western blot with the STI eluted and pooled fractions is shown in FIG. 10B. Molecular marker is loaded into lanes 1 and 1A; FXa is loaded into lanes 2 and 2B; and r-Antidote eluted from the STI-agarose column is loaded into lanes 4 and 4B of FIG. 10B.

**FIG. 11** shows the elution profile with a benzamidine gradient as described in Example 2.

**FIG. 12** shows the elution profile of the scale-up procedure described in Example 2.

**FIG. 13** shows the r-Antidote purification scheme for 25L medium (wave bags). UF = Ultrafiltration; MWCO = Molecular weight cut-off; UF/DF = Ultrafiltration/Diafiltration.

**FIG. 14** depicts a SDS-PAGE of purified r-Antidote as described in Example 2.

**FIG. 15** shows the elution profile of an STI-affinity column using an arginine elution buffer as described in Example 3.

**FIG. 16** depicts a reduced silver stained gel with loaded eluent from various STI-affinity columns with different STI-resins (A-E) as described in Example 3. The elution with arginine buffer yielded similar product profile as elution with benzamidine.

## DETAILED DESCRIPTION

### Definitions

The practice of the present disclosure will employ, unless otherwise indicated, conventional techniques of tissue culture, immunology, molecular biology, microbiology, cell biology and recombinant DNA, which are within the skill of the art. See, e.g., Sambrook et al., (1989) Molecular Cloning: A Laboratory Manual, 2nd edition; Ausubel et al., eds. (1987) Current Protocols In Molecular Biology; MacPherson, B.D. Hames and G.R. Taylor eds., (1995) PCR 2: A Practical Approach; Harlow and Lane, eds. (1988) Antibodies, A Laboratory Manual; Harlow and Lane, eds. (1999) Using Antibodies, a Laboratory Manual; and R.I. Freshney, ed. (1987) Animal Cell Culture.

As used herein, the term "about" generally means the stated value plus or minus a range of 10% of that value.

As used in the specification and claims, the singular form "a," "an" and "the" include plural references unless the context clearly dictates otherwise.

The term "protein", "peptide" and "polypeptide" are used interchangeably and in their broadest sense to refer to a compound of two or more subunit amino acids, amino acid analogs or peptidomimetics. The subunits may be linked by peptide bonds. In another embodiment, the subunit may be linked by other bonds, e.g., ester, ether, etc. A protein or peptide must contain at least two amino acids and no limitation is placed on the maximum number of amino acids which may comprise a protein's or peptide's sequence. As used herein the term "amino acid" refers to either natural and/or unnatural or synthetic amino acids, including glycine and both the D and L optical isomers, amino acid analogs and peptidomimetics. "Peptidomimetics" are small protein-like chains designed to mimic a peptide. They can be made from modification of an existing peptide, or by designing similar systems that mimic peptides, such as peptoids and β-peptides.

The term "isolated" or "recombinant" as used herein with respect to nucleic acids, such as DNA or RNA, refers to molecules separated from other DNAs or RNAs. The term "isolated" is also used herein to refer to polynucleotides, polypeptides and proteins that are isolated from other cellular proteins and is meant to encompass both purified and recombinant polypeptides. For example, an isolated cell is a cell that is separated from tissue or cells of dissimilar phenotype or genotype. An isolated polynucleotide is separated from the 3' and 5' contiguous nucleotides with which it is normally associated in its native or natural environment, e.g., on the chromosome. As is apparent to those of skill in the art, a non-naturally occurring polynucleotide, peptide, polypeptide, protein, antibody or fragment(s) thereof, does not require "isolation" to distinguish it from its naturally occurring counterpart.

The term "biological equivalent of" a protein, peptide or polynucleotide refers to one that has at least about 80 % homology or sequence identity and alternatively, at least about 85 %, or alternatively at least about 90 %, or alternatively at least about 95 %, or alternatively 98 % percent homology or sequence identity, and exhibits substantially equivalent biological activity to the reference protein, polypeptide or nucleic acid.

"Hybridization" refers to hybridization reactions that can be performed under conditions of different "stringency". Conditions that increase the stringency of a hybridization reaction are widely known and published in the art: see, for example, Sambrook, et al., infra. Examples of relevant conditions include (in order of increasing stringency): incubation temperatures of 25 °C, 37 °C, 50 °C, and 68 °C; buffer concentrations of 10 X SSC, 6 X SSC, 1 X SSC, 0.1 X SSC (where SSC is 0.15 M NaCl and 15 mM citrate buffer) and their equivalent using other buffer systems; formamide concentrations of 0%, 25%, 50%, and 75%; incubation times from 5 minutes to 24 hours and washes of increasing duration, increasing frequency, or decreasing buffer concentrations.

A polynucleotide or polynucleotide region (or a polypeptide or polypeptide region) having a certain percentage (for example, 80%, 85%, 90%, 95%, 97%, 98%, or 99%) of "sequence identity" to another sequence means that, when aligned, that percentage of bases (or amino acids) are the same in comparing the two sequences. The alignment and the percent homology or sequence identity can be determined using software programs known in the art, for example those described in Current Protocols in Molecular Biology (Ausubel et al., eds. 1987) Supplement 30, section 7.7.18, Table 7.7.1. Preferably, default parameters are used for alignment. A preferred alignment program is BLAST, using default parameters. In particular, preferred programs are BLASTN and BLASTP, using the following default parameters: Genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + SwissProtein + SPupdate + PIR. Details of these programs can be found at the following Internet address: ncbi.nlm.nih.gov/cgi-bin/BLAST.

"Homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares, for example, less than 40% identity, or alternatively less than 25% identity, with one of the sequences of the present disclosure.

The term "fraction" when used in the context of protein isolation, refers to a collection of material separated based on a specific property. The specific property may include, by way of non-limiting example, size, mass, isolectric point, charge, and the like.

"Factor Xa" or "fXa" or "fXa protein" refers to a serine protease in the blood coagulation pathway, which is produced from the inactive factor X (fX). Factor Xa is activated by either factor IXa with its cofactor, factor VIIIa, in a complex known as intrinsic Xase, or factor VIIa with its cofactor, tissue factor, in a complex known as extrinsic Xase. fXa forms a membrane-bound prothrombinase complex with factor Va and is the active component in the prothrombinase complex that catalyzes the conversion of prothrombin to thrombin. Thrombin is the enzyme that catalyzes the conversion of fibrinogen to fibrin, which ultimately leads to blood clot formation.

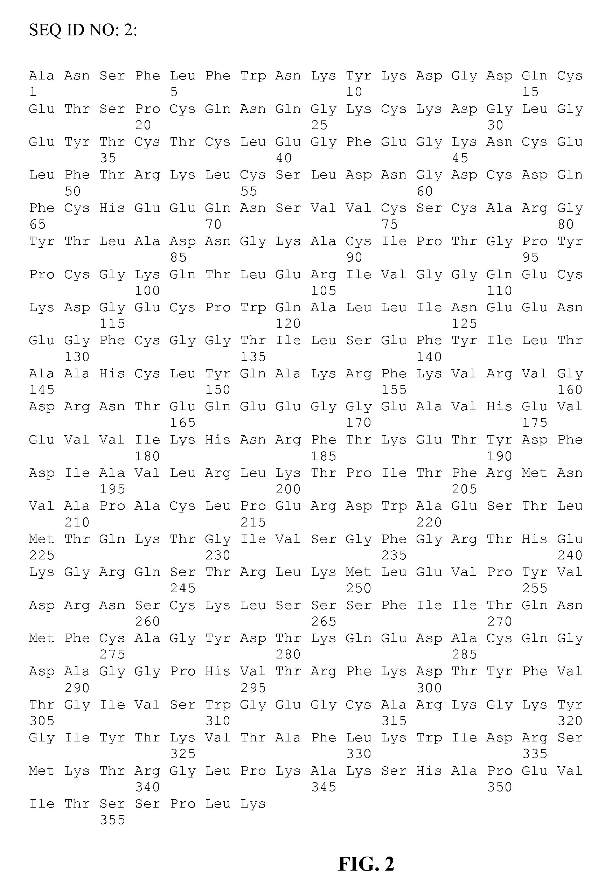

As used herein, a "fXa derivative" refers to a modified fXa protein that does not compete with fXa in assembling into the prothrombinase complex and yet binds and/or substantially neutralizes an anticoagulant, such as a fXa inhibitor. In some embodiments, the fXa derivative has reduced or no procoagulant activity. An example of a fXa derivative is provided herein as SEQ ID NO: 2 (**FIG. 2**) or a biological equivalent thereof.

The term "active site" refers to the part of an enzyme or antibody where a chemical reaction occurs. A "modified active site" is an active site that has been modified structurally to provide the active site with increased or deceased chemical reactivity or specificity. Examples of active sites include, but are not limited to, the catalytic domain of human factor X comprising the 235-488 amino acid residues, and the catalytic domain of human factor Xa comprising the 195-448 amino acid residues of fXa. Examples of modified active site include, but are not limited to, the catalytic domain of human factor Xa with at least one amino acid substitution at position Arg306, Glu310, Arg347, Lys351, Lys414, or Arg424.

As stated above, the derivatives of the invention may have modified Gla domains or have the entire Gla domain removed. The Gla domain of the fXa refers to amino acids residues 1-45 of the wild-type fXa protein. In some embodiments, the derivatives have a complete (1-45) or partial deletion of the Gla domain (e.g., 1-44, 1-39, or 6-39).

"r-Antidote precursor" refers to a fXa derivative represented by SEQ ID NO: 1, which contains three mutations relative to human wild-type fXa. The first mutation is a deletion in the Gla-domain of the wild-type fX protein at position 6-39. The second mutation is replacing the activation peptide sequence 143-194 amino acids with -RKR-. This produces a -RKRRKR- (SEQ ID NO: 3) linker connecting the light chain and the heavy chain. Upon secretion, this linker is cleaved in CHO resulting in a cleaved two-chain polypeptide. The term "cleaved two-chain polypeptide" refers to a polypeptide of SEQ ID NO: 2, or a polypeptide having 80% identity to SEQ ID NO: 2, having two-chains and being linked together by a disulfide bond. The N-terminal chain consist of amino acids 1-105 of SEQ ID NO: 2 and the C-terminal chain consists of amino acids 106-359 of SEQ ID NO: 2. Optionally, the LC chain may contain 1, 2, 3, 4, 5 or 6 amino acid residues of the linker. Such additional residues result from the incomplete removal of the linker polypeptide. The third mutation is a mutation of active site residue S379 to an Ala residue (amino acid numbering based on secreted human fX sequence). This amino acid substitution corresponds to amino acid 296 and 290 of SEQ ID NOS: 1 and 2, respectively.

The term "r-Antidote" may refer to the polypeptide before removal of the linker (SEQ ID NO: 1) or after removal of the linker (SEQ ID NO: 2). U.S. Application 13/766,652, describes methods and cells for the improved or enhanced processing of one-chain r-Antidote precursor to cleaved two-chain r-Antidote protein that acts as an antidote to fXa inhibitors.

"STI" or "Soybean Trypsin Inhibitor," refers to trypsin inhibitors isolated from soybeans, or their biological equivalents. Trypsin inhibitors are about 20 kDa in size and reduce trypsin (a proteolytic enzyme) as well as plasma kallikrein, factor Xa and plasmin activity. STI are commercially available from vendors such as Life Technologies (Grand Island, NY). An example of STI is KTI3 Kunitz trypsin inhibitor, from Glycine max (soybean), having a GenBank accession number NP_001238611.

The term "competitive agent" is a molecule that can aid in the elution of the serine protease from the STI affinity column either by disruption of a charge-charge interaction between the STI and the serine protease or by competing with STI for binding to the serine protease. Non-limiting examples include benzamidine, p-aminobenzamidine, arginine, a small molecule fXa inhibitor, a peptide fXa inhibitor, and a peptidomimetic fXa inhibitor.

The term "factor Xa inhibitors" or "inhibitors of factor Xa" refer to compounds, peptides, peptidemimetics, that can inhibit, either directly or indirectly, the coagulation factor Xa's activity of catalyzing conversion of prothrombin to thrombin *in vitro* and/or *in vivo.* Examples of known fXa inhibitors include, without limitation, fondaparinux, idraparinux, biotinylated idraparinux, enoxaparin, fragmin, NAP-5, rNAPc2, tissue factor pathway inhibitor, DX-9065a (as described in, e.g., Herbert, J.M., et al, J Pharmacol Exp Ther. 1996 276(3):1030-8), YM-60828 (as described in, e.g., Taniuchi, Y., et al, Thromb Haemost. 1998 79(3):543-8), YM-150 (as described in, e.g., Eriksson, B.I. et. al, Blood 2005;106(11), Abstract 1865), apixaban, rivaroxaban, PD-348292 (as described in, e.g., Pipeline Insight: Antithrombotics - Reaching the Untreated Prophylaxis Market, 2007), otamixaban, razaxaban (DPC906), BAY 59-7939 (as described in, e.g., Turpie, A.G., et al, J. Thromb. Haemost. 2005, 3(11):2479-86), DU-176b (as described in, e.g., Hylek EM, Curr Opin Invest Drugs 2007 8(9):778-783), LY517717 (as described in, e.g., Agnelli, G., et al, J. Thromb. Haemost. 2007 5(4):746-53), GSK913893, betrixaban (as described below) and derivatives thereof. Low molecular weight heparin ("LMWH") is also considered a factor Xa inhibitor.

The term "chaotropic agent" intends a substance which disrupts the structure of, and denatures, macromolecules such as proteins and nucleic acids. Chaotropic agents include, for example, butanol, ethanol, guanidinium chloride, lithium perchlorate, magnesium chloride, phenol, propanol, sodium dodecyl sulfate, thiourea, and urea.

## Methods

The present disclosure describes methods for purifying serine proteases in active form from a sample containing the serine proteases. These methods are superior to conventional methods for the purification of serine proteases. Further, the methods are effective in the purification of even modified serine proteases, which modifications, such as those in r-Antidote as compared to the wild-type fXa, affect the proteases' binding and/or enzymatic activities.

It has been shown that factor Xa and other serine proteases can be purified using a benzamidine-Sepharose affinity chromatographs. Initial tests (see for e.g. Example 1) indicate that certain fXa derivatives lacking the Gla-domain have low affinity to conventional ion-exchange chromatographs such as Q-Sepharose due to deletion of the Gla-domain. Surprisingly, r-Antidote does not bind to benzamidine-Sepharose affinity chromatograph, or other similar commercially available affinity chromatograph of small ligands. The present disclosure describes the purification of serine proteases using a STI-based affinity chromatograph and an elution step with a competitive agent which is arginine, and optionally a salt and a detergent.

The serine protease can be recombinantly produced as previously described, e.g., in 13/766,652, or by other methods of recombinant protein production known in the art. For example, proteins may be cloned into a DNA construct (i.e. plasmids, viral vectors, cosmids, expression vectors, phagemids, fosmids, and artificial chromosomes such as bacterial artificial chromosomes, yeast artificial chromosomes, and human artificial chromosomes) and introduced into a suitable host cell by gene transfer techniques such chemical-based transfection, such as calcium phosphate transfection and polyfection, and non chemical-based transfection such as electroporation, optical transfection, and gene electrotransfer. Suitable host cells include prokaryotic and eukaryotic cells, which include, but are not limited to bacterial cells, yeast cells, insect cells, animal cells, mammalian cells, murine cells, rat cells, sheep cells, simian cells and human cells. Cells can then be lysed by physical techniques such as sonication or freeze-thaw or by the use of detergents or lysis buffers such as RIPA Buffer (Radi-Immunoprecipitation Assay) containing 150 mM NaCl, 1.0% IGEPAL® CA-630, 0.5% sodium deoxycholate, 0.1% SDS, and 50 mM Tris, pH 8.0, or by physical separation, such as centrifugation or filtration, to obtain the clarified harvested culture fluid from mammalian cell cultures. The resulting soluble protein extract may be then used in the purification methods described herein.

The protein extract is applied to a Soybean Trypsin Inhibitor (STI)-based affinity chromatographs. Soybean Trypsin Inhibitor is a 20 kDa protein and can be immobilized on a solid support resin for the purification of certain proteins. In addition to Soybean Trypsin Inhibitor, other trypsin inhibitor proteins such as those isolated from serum, lima beans, bovine pancreas, or ovomucoid, or the modified forms thereof can also be used. It is also contemplated that certain protease inhibitors, in particular serine protease inhibitors, can be used to prepare affinity resin for the purpose of purifying the r-Antidote, serine proteases or other Factor Xa derivatives. Identification of suitable ones from these protease inhibitors can be carried out with methods disclosed herein.

The serine protease may then be eluted with an elution buffer comprising a competitive agent, a salt, a detergent, or a chaotropic agent. The competitive agent interrupts the interaction between the STI and the serine protease or by competing with STI for binding to the serine protease. Non-limiting examples of competitive agents include benzamidine, arginine, small molecule fXa inhibitors, peptide fXa inhibitors, or peptidomimetic fXa inhibitors. Direct factor Xa inhibitors include NAP-5, rNAPc2, tissue factor pathway inhibitor, DX-9065a, YM-60828, YM-150, apixaban, rivaroxaban, TAK-442, PD-348292, otamixaban, edoxaban, LY517717, GSK913893, razaxaban, betrixaban or a pharmaceutically acceptable salt thereof, and combinations thereof. Peptide inhibitors of fXa include the tripeptide keto thiazole and the corresponding aldehyde as described in Betz et al., "Inhibition of Factor Xa by a Peptidyl-$\alpha$-ketothiazole Involves Two Steps. Evidence for a Stabilizing Conformational Change," Biochemistry (1999), 38, 14582-14591.

The competitive agent is arginine. Elution with arginine is advantageous because it is a GRAS (Generally Recognized As Safe) excipient and does not need to be removed from the purified protein. An additional benefit of arginine is that it actually improves the solubility of a serine protease (e.g., r-Antidote) and can be used as an excipient in the final formulation.

The concentration of arginine employed in the elution buffer may be from about 250 mM to about 1000 mM. In one embodiment, the concentration of arginine in the elution buffer is about 500 mM. In further embodiments, the concentration is about 250 mM, or about 300 mM, or about 350 mM, or about 400 mM, or about 450 mM, or about 550 mM, or about 600 mM, or about 650 mM, or about 700 mM, or about 750 mM, or about 800 mM, or about 850 mM, or about 900 mM, or about 1 M. The elution buffer optionally further comprises a salt, a detergent, or a chaotropic agent. Salts useful in the elution buffer of the methods and kits disclosed herein include sodium chloride, ammonium chloride, sodium citrate, potassium citrate, potassium chloride, magnesium chloride, calcium chloride. sodium phosphate. calcium phosphate, ammonium phosphate, magnesium phosphate, potassium phosphate, sodium sulfate, ammonium sulfate, potassium sulfate, magnesium sulfate, calcium sulfate, etc. Detergents useful in the elution buffer of the methods and kits disclosed herein include, for example, polysorbate 80, urea, guanidine, etc.

The pH of the elution buffer in the methods and kits described herein is one that allows for the effective elution of a serine protease protein absorbed on the resin without causing inactivation and/or precipitation of the serine protease. Certain fXa derivatives such as r-Antidote are inactivated or precipitate at low pH. In certain embodiments, the pH of the elution buffer is from about 4.5 to about 10.5. In another embodiment, the pH of the elution buffer is about pH 5.0. In another embodiment, the pH of the elution buffer is about pH 7.4. Alternatively, the pH of the elution buffer is at least about 4.5, about 5.5, about 6, about 6.5, about 7, about 7.5, about 8.0, about 8.5, about 9, about 9.5, or at least about 10. In another embodiment, the pH of the elution buffer is not higher than about 5.5, about 6, about 6.5, about 7, about 7.5, about 8.0, about 8.5, about 9, about 9.5, about 10, or not higher than about 10.5. In one embodiment, the pH of the elution buffer is about pH 5.0 when arginine is used as the competitive agent.

In certain embodiments, the serine protease is a fXa derivative which, for example, is a polypeptide comprising the amino acid sequence of SEQ ID NO: 1 or 2 or a polypeptide having at least about 80%, 85%, 90%, 95%, 98%, or 99% sequence identity to SEQ ID NO: 1 or 2. In some embodiments, the polypeptide having sequence identity to SEQ ID NO: 1 or 2 has substantially the same activity as SEQ ID NO: 1 or 2. The fXa derivative represented by SEQ ID NO: 1 contains three mutations relative to fXa. The first mutation is the deletion in the Gla-domain of FX at position 6-39 in the wild-type protein. The second mutation replaces the activation peptide sequence 143-194 aa with -RKR-. This produced a - RKRRKR- (SEQ ID NO: 3) linker connecting the light chain and the heavy chain. Upon secretion, this linker is cleaved in CHO resulting in a two-chain fXa molecule (SEQ ID NO: 2). The third mutation is mutation of active site residue S379 to an Ala residue (based on secreted human fX amino acid sequence). This amino acid substitution corresponds to amino acid at position 296 and position 290 of SEQ ID NOS: 1 and 2, respectively. The fXa derivative does not compete with fXa in assembling into the prothrombinase complex, but instead bind and/or substantially neutralize the anticoagulants, such as fXa inhibitors. The derivatives useful as antidotes are modified to reduce or remove intrinsic procoagulant and anticoagulant activities, while retaining the ability to bind to the inhibitors. Structurally, the derivatives are modified to provide either no procoagulant activity or reduced procoagulant activity. "Procoagulant activity" is referred to herein as an agent's ability to cause blood coagulation or clot formation. Reduced procoagulant activity means that the procoagulant activity has been reduced by at least about 50%, or more than about 90%, or more than about 95% as compared to wild-type fXa. In a related embodiment, the amino acid sequence having at least 80% sequence identity to SEQ ID NO: 2 has reduced procoagulant activity compared to wild-type factor Xa. In a further embodiment, the amino acid sequence having at least 80% sequence identity to SEQ ID NO: 2 does not assemble into a prothrombinase complex.

In one embodiment, the serine protease may be added (loaded) to the column under conditions that allow for the absorption of the serine protease on to the column, and the column may be washed with a wash buffer that allows for the continued absorption of the serine protease to the column and contaminating proteins or molecules in the flow-through. In one embodiment, the wash buffer may comprise a salt and be at a neutral pH. The term "neutral pH" is intended to mean a pH from about 6 to about 8. In certain embodiments, the wash buffer comprises from about 200 to about 500 mM NaCl at a neutral pH. In other embodiments, the pH is about 6, or about 7, or about 8.

The methods disclosed herein may further comprise other purification and chromatographic steps such as, for example, filtration, ion-exchange chromatography, mixed-mode resins, exclusion chromatography, polyacrylamide gel electrophoresis, affinity chromatography, or isoelectric focusing. In one embodiment, the method further comprises applying the solution containing the polypeptide to an ion-exchange column.

Suitable cation-exchange resins include a wide variety of materials known in the art, including those capable of binding polypeptides over a wide pH range. For example, carboxymethylated, sulfonated, agarose-based, or polymeric polystyrene/divinyl benzene cation-exchange matrices are particularly preferred. Other useful matrix materials include, but are not limited to, cellulose matrices, such as fibrous, microgranular, and beaded matrices; dextran, polyacrylate, polyvinyl, polystyrene, silica, and polyether matrices; and composites. Other suitable materials for use in cation exchange chromatography are within the knowledge of those skilled in the art.

Anion-exchange chromatography is carried out using media appropriate therefor, as are known in the art. Suitable media include, e.g., polymeric polystyrene/divinyl benzene resins and agarose-based resins, as well as agarose beads, dextran beads, polystyrene beads, media that comprise an insoluble, particulate support derivatized with tertiary or quaternary amino groups., and supports derivatized with trimethylaminoethyl groups. Examples of suitable such media include DE92 (diethylaminoethyl cellulose, Whatman); DEAE CELLULOSE (Sigma), BAKERBOND ABX 40 mu (J. T. Baker, Inc.); DEAE resins such as FRACTOGEL EMD DEAE-650 (EM Separations), FRACTOGEL EMD TMAE-650 (S) TM (EM Science, Gibbstown, NJ), TSK gel DEAE-SPW (Tosohaas), DEAE-SEPHAROSE CL-6BT"and chelating SEPHAROSE (Amersham Pharmacia Biotech AB), DEAE MERE SEP. IOOOTM (Millipore), and DEAE SPHERODEX (Sepracor); RESOURCE QTm and Q SEPHAROSE (QSFF) (Amersham Pharmacia Biotech AB); MACRO-PEP QTM (Bio-Rad Laboratories, Hercules, CA); Q-HYPERD (BioSepra, Inc., Marlborough, Mass); and the like. Other suitable anion-exchange chromatography materials, as well as the selection and use of these materials for the present application, are conventional in the art.

The ion-exchange chromatography, filtration, nanofiltration, or additional purification step may be prior to or after the STI-affinity chromatography. Additional steps may also include viral inactivation steps by, for example, solvent and detergent treatment of the protein extract or through nanofiltration.

Generally, "purification" refers to increasing the concentration and/or purity of a protein or peptide in a sample. In some embodiments, the purification process subjects the sample to fractionation to remove various other components, during which the protein or peptide substantially retains its biological activity. A substantially purified protein or peptide in a composition forms the major component of the composition, such as constituting at least about 50%, at least about 60%, at least about 70%, at least about 80%, at least about 90%, at least about 95% or more of the proteins in the dry components of a sample solution.

Various methods for quantifying the degree of purification of the protein or peptide will be known to those of skill in the art in light of the present disclosure. These include, for example, determining the specific activity of an active fraction, or assessing the amount of polypeptides within a fraction by SDS/PAGE analysis. A preferred method for assessing the purity of a fraction is to calculate the specific activity of the fraction, to compare it to the specific activity of the initial extract, and to thus calculate the degree of purity, herein assessed by a "-fold purification number." The actual units used to represent the amount of activity will, of course, be dependent upon the particular assay technique chosen to follow the purification and whether or not the expressed protein or peptide exhibits a detectable activity.

Various techniques suitable for use in protein purification will be well known to those of skill in the art. These include, for example, precipitation with ammonium sulfate, PEG (polyethylene glycol), antibodies and the like or by heat denaturation, followed by centrifugation; chromatography steps such as ion exchange, gel filtration, reverse phase, hydroxylapatite and affinity chromatography; isoelectric focusing; gel electrophoresis; and combinations of such and other techniques.

A further aspect disclosed herein relates to a purified serine protease comprising the amino acid sequence of SEQ ID NO: 1 or 2 or a polypeptide having at least about 80%, at least about 85%, at least about 90%, at least about 95%, at least about 97%, at least about 98%, or at least about 99% sequence identity to SEQ ID NO: 1 or 2 wherein the polypeptide is produced by the methods described herein. In some embodiments of the disclosure, the polypeptide having sequence identity to SEQ ID NO: 1 or 2 has substantially the same activity as SEQ ID NO: 1 or 2.

The present application also describes a kit comprising a soybean trypsin inhibitor affinity resin; an elution buffer comprising arginine; and instructions for use. In one embodiment, the kit further comprises a wash buffer. In a related embodiment, the wash buffer comprises about 250 mM NaCl at a neutral pH.

## EXPERIMENTAL EXAMPLES

The disclosure is further understood by reference to the following examples, which are intended to be purely exemplary of the disclosure.

Unless otherwise stated all temperatures are in degrees Celsius. Also, in these examples and elsewhere, abbreviations have the following meanings:

| | |
|---|---|
| hr | = hour |
| cm | = centimeter |
| L | = liter |
| M | = molar |
| mg | = milligram |
| mg/kg | = milligram/kilogram |
| mg/mL | = milligram/milliliter |
| min | = minute |
| mL | = milliliter |
| $\mu$L or uL | = microliter |
| $\mu$M | = micromolar |
| fX | = factor X |
| fXa | = factor Xa |
| HC | = heavy chain |
| LC | = light chain |
| MTX | = methotrexate |
| PBS | = phosphate buffered saline |
| STI | = soybean trypsin inhibitor |

## EXAMPLE 1

### Screening of Commercially Available Affinity Resins

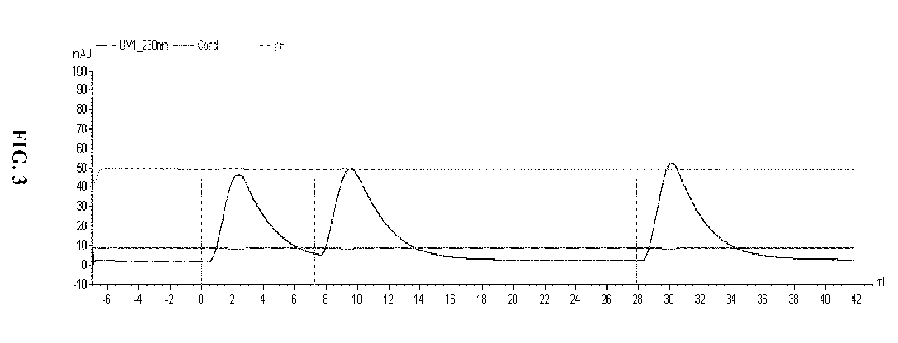

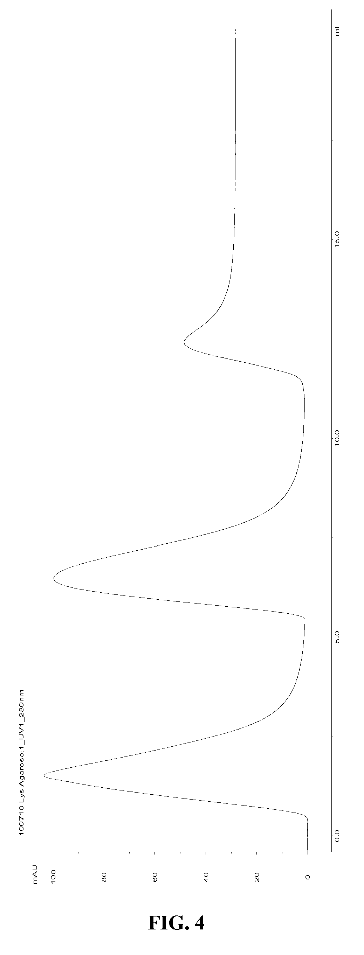

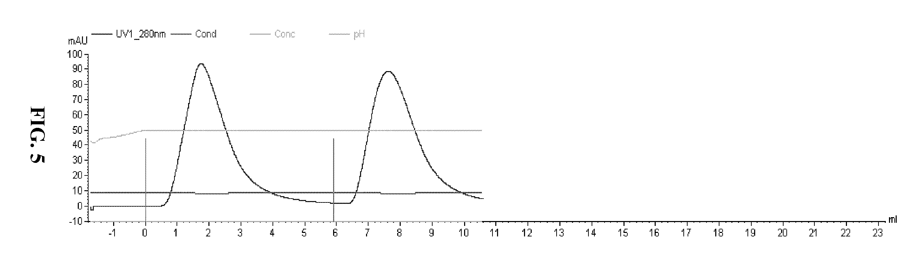

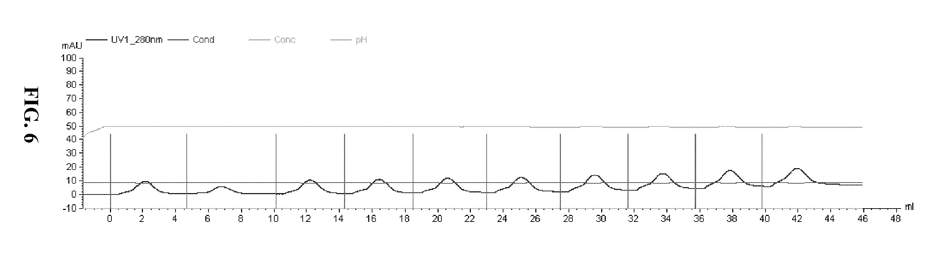

Commercially available affinity resins were screened for their efficacy in purifying r-Antidote. Various affinity resins were packed into a column (such as a Tricorn column, GE) with 1 mL of resin. Following sanitization according to manufacturer's recommendations, the column was equilibrated with 20 mM Tris/150 mM NaCl at pH 7.4, followed by testing their ability to bind r-Antidote in the same buffer. r-Antidote at a concentration of 0.145 mg/mL in PBS (Phosphate Buffered Saline), was loaded onto the column at a flow rate of 1 mL/min by injection (1mL). Surprisingly, no binding of r-Antidote to the Benzamidine-Agarose (catalog# A7155, Sigma) (FIG. 3), L-Lysine-Agarose (Catalog# L5631) (FIG. 4), and Aprotinin-Agarose (catalog# A2268) (FIG. 5) affinity columns was observed. In contrast, r-Antidote was found to bind to the STI-affinity resin (FIG. 6).

**EXAMPLE 2**

**Purification of r-Antidote by the STI-Affinity Resin with Elution Buffer Containing Benzamidine**

The harvested cell culture conditioned media containing the r-Antidote was first passed through a Q-Sepharose fast flow (catalog# 17-0510-01, GE Healthcare) pre-column buffered in a solution of 20 mM Tris and 250 mM NaCl at pH 7.4. The flow-through from the pre-column containing the r-Antidote was then applied to a STI-Agarose (Sigma, T-0635) column under conditions that allowed for the absorption of the r-Antidote to the column. The column was washed with a solution containing 20 mM Tris and 250 mM NaCl at pH 7.4 (1X Q-BF). The r-Antidote was then eluted with an elution buffer containing 20 mM Tris, 150 mM NaCl, at pH 7.4 with either 0.5 M or 1.0 M benzamidine. The STI-Agarose capacity based on the commercial data insert is 1-3 mg/mL trypsin.

The cell culture media used in the various tests described in this example was produced from a CHO-DUXB 11 clone (designated as 1C2) which was selected under 50 nM methotrexate (MTX). Prior to loading to the STI-Agarose column, the cell culture media was dialyzed into the same buffer used for the Q-Sepharose pre-column (20 mM Tris and 250 mM NaCl at pH 7.4).

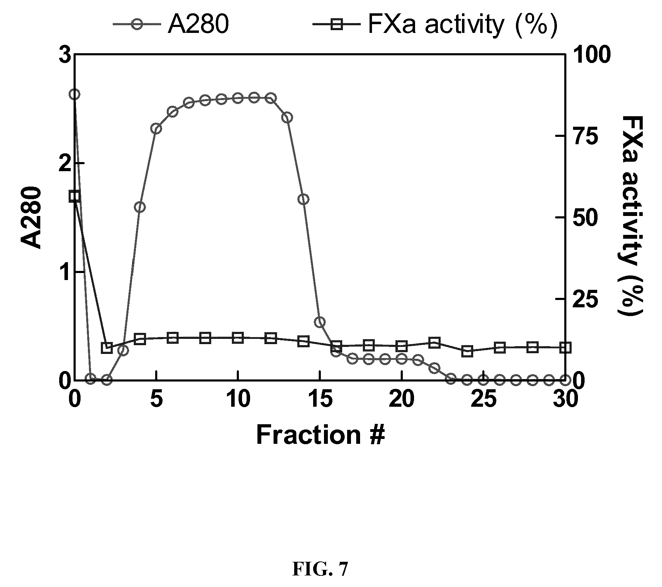

When 50 mL of dialyzed cell culture medium was loaded directly on to STI-Agarose affinity column, the functional r-Antidote was captured by the STI-resin (FIG. 7). No functional r-Antidote was detected in the flow-through fractions (NOTE: fXa chromogenic activity assay description is needed. Can be copied from r-antidote application). The r-Antidote function was detected by using a fXa chromogenic activity assay in the presence of a fXa inhibitor. r-Antidote is able to bind to the inhibitor and thus reduces its inhibitory activity. The FXa Activity (%) was calculated by:

$$\text{FXa Activity (\%)} = \text{fXa activity (with inhibitor)}/\text{fXa activity (no inhibitor)} \times 100.$$

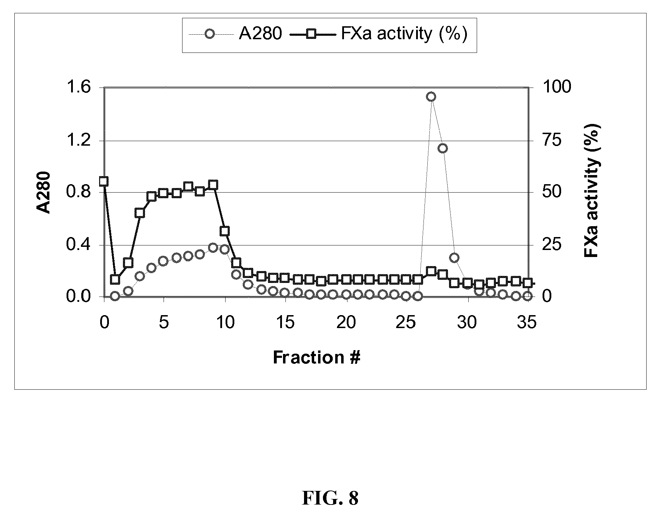

Unlike the STI-Agarose resin just described above (FIG. 7), when the same dialyzed cell culture media (50 mL) was loaded on a HQ-Sepharose pre-column (125 mL), the target protein is, however, in the flow-through fractions (FIG. 8). The HQ-Sepharose pre-column was washed with 1X Q-BF until fraction 25. The wash buffer was switched to 20 mM Tris, 1 M NaCl at pH 7.4 at fraction 25. The HQ-Sepharose flow-through fractions were pooled based on fXa functional activity assay (total 475 mL). This result demonstrates that HQ-Sepharose could be used in a flow-through mode for r-Antidote purification, either as a pre-column used prior to the STI-Agarose affinity column or as a follow on step after the STI-Agarose affinity column.

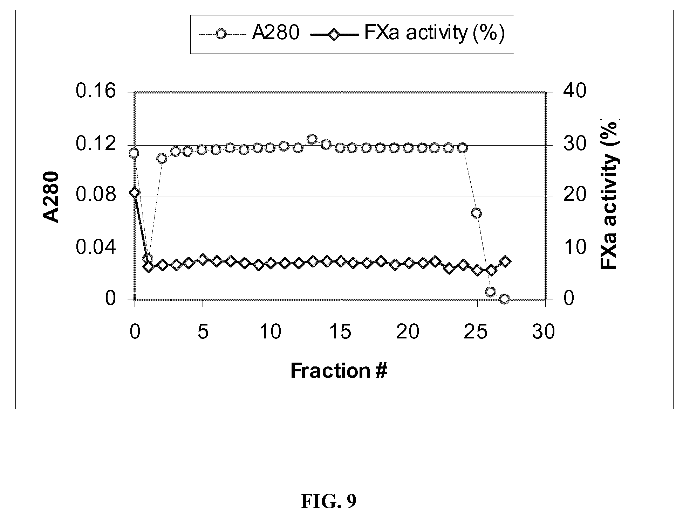

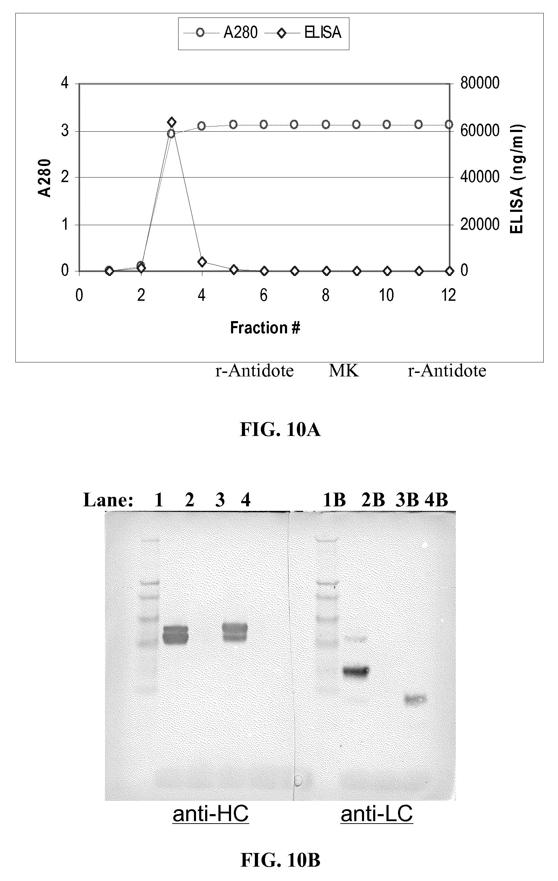

The pooled fractions from the HQ-Sepharose flow-through fractions were loaded onto a STI-Agarose affinity resin (10 mL), and the resin was then washed with 1X Q-BF. FIG. 9 demonstrates that the r-Antidote was absorbed onto the STI-affinity resin. The bound proteins were then eluted with an elution buffer of 20 mM Tris, 150 mM NaCl at pH 7.4, and 1M benzamidine. FIG. 10A shows the elution profile with the benzamidine elution buffer. Because benzamidine in the elution buffer interferes with the fXa functional activity assay, r-Antidote in the elution fractions was quantified by an enzyme-linked immunosorbent assay (ELISA) with a commercially available paired-antibodies recognizing human fX/fXa (FX-EIA, Enzyme Research Laboratories Inc.). The r-Antidote-containing fractions were pooled and benzamidine was removed by dialysis with PBS.

The affinity purified r-Antidote was analyzed by Western blot (FIG. 10B) using antibodies recognizing specifically the heavy chain (HC) and light chain (LC) of human fX/fXa (Enzyme Research Laboratories Inc.). FIG. 10B shows that r-Antidote is eluted with benzamidine.

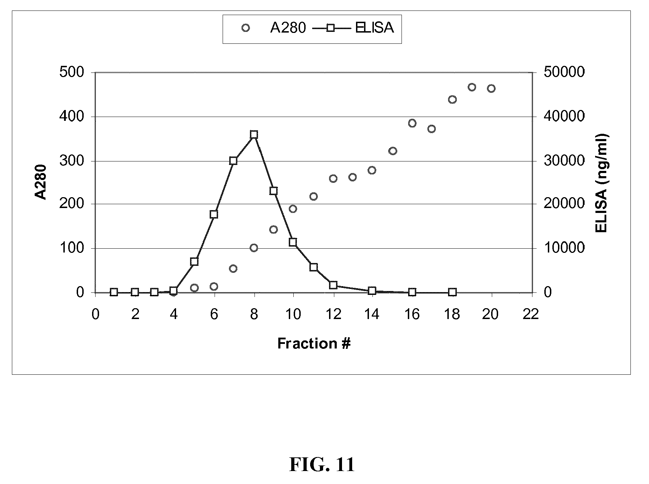

In order to further determine the benzamidine concentration which is required to elute the r-Antidote from the STI-Agarose resin, the elution of the r-Antidote from the STI-Agarose column was also performed using a benzamidine gradient (FIG. 11).

The cell culture media was first loaded on a 1 mL STI-Agarose column and washed as previously described (FIG. 7), the bound r-Antidote was eluted by a gradient comprising of 10 column volume (CV) of Buffer A and Buffer B.. Buffer A (20 mM Tris, 150 mM NaCl at pH 7.4), and buffer B consisted of 20 mM Tris, 150 mM NaCl at pH 7.4 and1M benzamidine. The benzamidine concentration (gradient) was estimated by measuring absorbance at 280 nm in each fraction and the r-Antidote was analyzed by ELISA, The target protein elution peak was at about 250 mM benzamidine and the target protein elution ended at about 500 mM benzamidine (FIG. 11). Therefore, the optimal benzamidine concentration in the elution buffer is about 500 mM.

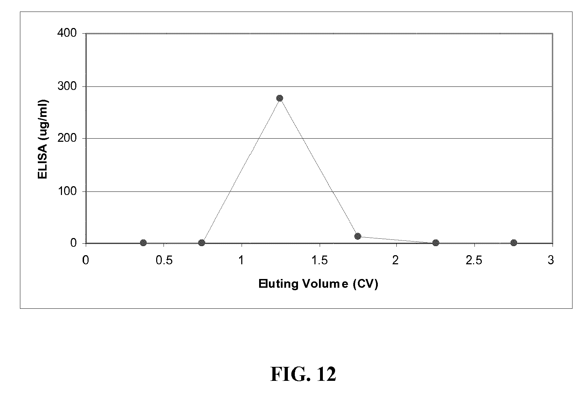

The purification procedure can be scaled-up to acquire larger amounts of product. For example, in order to purify the r-Antidote from 10 L of harvested cell culture fluid, 500 mL of a HQ-Sepharose column may first be used, followed by 200 mL of STI-Agarose. The elution in the scale-up procedure was done with 1 M benzamidine in 1X Q-buffer (FIG. 12).

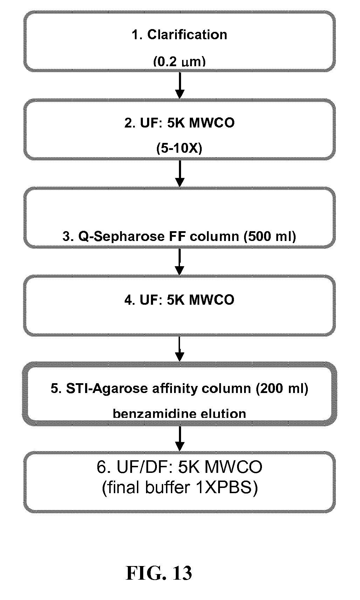

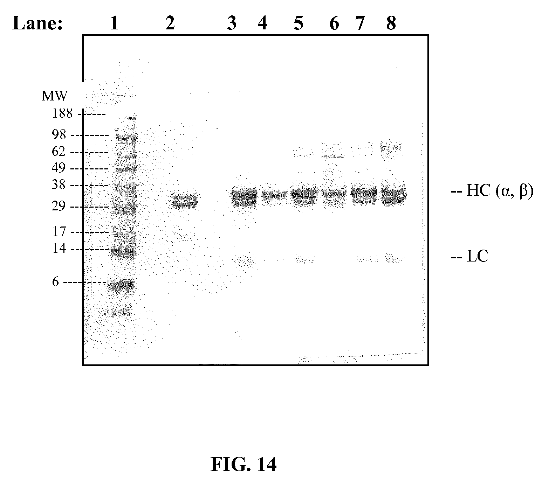

Thus, above findings may be integrated into a purification scheme (FIG. 13) for r-Antidote purification from various batches of cell culture fluid (10 - 25 L). To determine the quality of purified r-Antidote in the scale-up scheme, 3.5 µg of purified protein was loaded into separate wells of a SDS-PAGE gel (10% bis-tris gel/MES buffer, reduced) (FIG. 14). The lanes were loaded with 1) Molecular weight marker; 2) FXa protein; 3) Lot 1 - 16 L; 4) Lot 2 - 8 L; 5) Lot 4 - First 25 L; 6) Lot 5 - second 25 L; 7) Lot 6 - third 25 L and 8) Lot 7 - fourth 25 L, with each lot number followed by the starting volume of cell culture fluid.

**EXAMPLE 3**

**Purification of r-Antidote by the STI-Affinity Resin with Elution Buffer Containing Arginine**

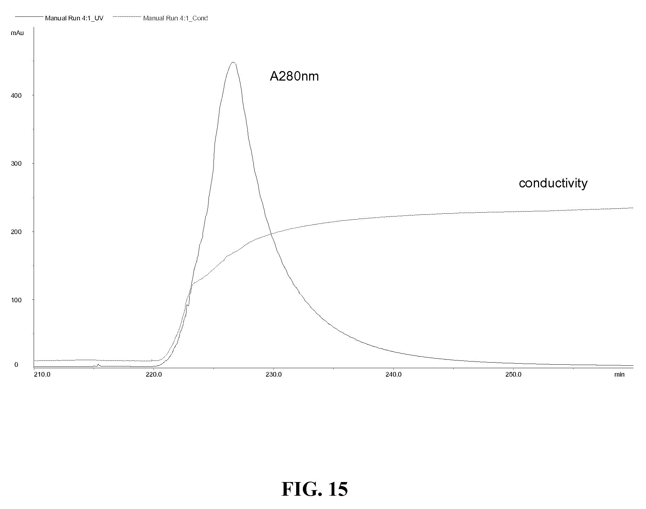

To test whether r-Antidote could be eluted with arginine, 5-10 mg r-Antidote protein extract from cell culture was loaded onto 1 mL STI-affinity column through a pump at 0.2 mL/min (about 61 cm/hr). The protein was eluted with 0.5 M arginine in 25 mM Na-acetate at pH 5.0 and the pH was immediately adjusted to about pH 7.4 by addition of appropriate volume of 1M Tris (pH 8.0). The column was then stripped with 1.0 M arginine, 25 mM Na-acetate at pH 5, followed by 0.5 M benzamidine, 20 mM Tris, and 150 mM NaCl at pH 7.4. FIG. 15 shows a representative elution profile with the arginine elution buffer. The elution buffer containing 0.5M arginine is able to elute the r-Antidote off the STI-Affinity resin. No additional r-Antidote protein was found in stripping fractions with either 1M arginine or 0.5M benzamidine.

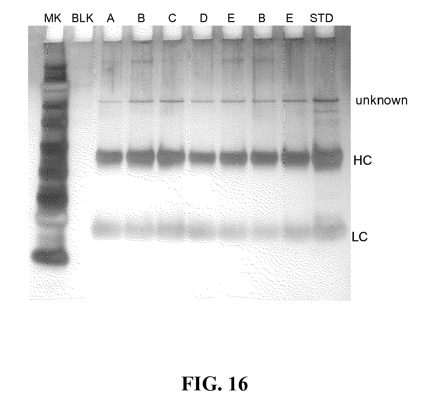

Furthermore, the elution with arginine buffer yielded similar product profile as elution with benzamidine (FIG. 16). Thus, it is discovered that a STI-affinity based resin with an arginine-containing elution buffer could be used to effectively purify the r-Antidote from the cell culture conditioned media.

In order to determine the binding capacities of various specifically modified - STI-affinity resins, the binding capacity of the resins (resins A-K) were tested under similar conditions (1mL column). The bound protein was eluted with the elution buffer containing 0.5M arginine. r-Antidote in elution fractions was quantified by both ELISA and absorbance at 280 nm. The binding capacities based on absorbance at 280 nm tabulated below.

| Example # | Resin # | Ligand Density (mg STI/mL resin) | Binding Capacity (mg r-Antidote/mL resin, protein measured by A280) |
|---|---|---|---|
| | | | |
| 1 | A | 3 | 2.4 |
| 2 | B | 5 | 5.1 |
| 3 | C | 7 | 6.2 |
| 4 | D | 3 | 2.5 |
| 5 | E | 4 | 2.9 |
| 6 | F | 6 | 5.3 |
| 7 | | | 5.8 |
| 8 | G | 5 | 4.7 |
| 9 | H | 5 | 3.1 |

| 10 | I | 5 | 4.9 |
| 11 | J | 4 | 3.1 |
| 12 | K | 4 | 4.1 |

## Claims

1. A method of purifying a serine protease comprising:

   loading the serine protease to a soybean trypsin inhibitor (STI)-based affinity chromatograph, and
   eluting the serine protease with an elution buffer comprising arginine that disrupts interaction between the STI
   and the serine protease.

2. The method of claim 1, wherein the elution buffer further comprises a salt, a detergent and/or a chaotropic agent.

3. The method of claim 1, wherein the serine protease is a polypeptide comprising the amino acid sequence of SEQ
   ID NO: 1 or 2, or a polypeptide having at least about 80% sequence identity to SEQ ID NO: 1 or 2, having a deletion
   of at least part of the Gla domain and a mutation at the active site.

4. The method of claim 3, wherein the serine protease comprises the amino acid sequence of SEQ ID NO: 2, or a polypeptide having at least about 95% sequence identity to SEQ ID NO: 2, having a deletion of at least part of the Gla domain and a mutation at the active site.

5. The method of claim 3, wherein the serine protease comprises the amino acid sequence of SEQ ID NO: 2.

6. The method of any one of claims 1-5, wherein the pH of the elution buffer is from about 4.5 to about 10.5.

7. The method of claim 6, wherein the pH of the elution buffer is about 5.0 or is about 7.4.

8. The method of claim 1, wherein the elution buffer comprises from about 250 mM arginine to about 1000 mM arginine.

9. The method of claim 8, wherein the elution buffer comprises about 500 mM arginine.

10. The method of claim 9, wherein the pH of the elution buffer is about 5.0.

11. The method of any one of claims 1-10, further comprising subjecting the eluted serine protease to purification with an ion-exchange column.

12. The method of any one of claims 1-11, further comprising, prior to eluting the serine protease, washing the chromatograph with a wash buffer that comprises a salt and is at a neutral pH.

13. A kit comprising:

   a soybean trypsin inhibitor (STI)-based affinity chromatograph, and
   an elution buffer comprising arginine that disrupts the interaction between the STI and a serine protease.

14. The kit of claim 13, wherein

   (i) the pH of the elution buffer is from about 4.5 to about 10.5; or
   (ii) the elution buffer comprises from about 250 mM arginine to about 1000 mM arginine.

15. The kit of claim 13 or 14, further comprising a wash buffer comprising about 250 mM NaCl at a neutral pH.

**Patentansprüche**

1. Verfahren des Reinigens einer Serinprotease, umfassend:

   Laden der Serinprotease auf einen Sojabohnentrypsininhibitor (STI)-basierten Affinitätschromatographen, und
   Eluieren der Serinprotease mit einem Arginin enthaltenden Elutionspuffer, der die Zwischenwirkung zwischen dem STI und der Serinprotease unterbricht.

2. Verfahren nach Anspruch 1, wobei der Elutionspuffer ferner ein Salz, ein Reinigungsmittel und/oder ein chaotropes Mittel umfasst.

3. Verfahren nach Anspruch 1, wobei die Serinprotease ein Polypeptid ist, das die Aminosäuresequenz SEQ ID NO: 1 oder 2 umfasst oder ein Polypeptid, das mindestens ca. 80 % Sequenzidentität mit SEQ ID NO: 1 oder 2 aufweist, mit einer Deletion zumindest eines Teils der Gla-Domäne und einer Mutation an der aktiven Stelle.

4. Verfahren nach Anspruch 3, wobei die Serinprotease die Aminosäuresequenz SEQ ID NO: 2 umfasst oder ein Polypeptid, das mindestens ca. 95 % Sequenzidentität mit SEQ ID NO: 2 aufweist, mit einer Deletion zumindest eines Teils der Gla-Domäne und einer Mutation an der aktiven Stelle.

5. Verfahren nach Anspruch 3, wobei die Serinprotease die Aminosäuresequenz SEQ ID NO: 2 umfasst.

6. Verfahren nach einem der Ansprüche 1 bis 5, wobei der pH-Wert des Elutionspuffers ca. 4,5 bis ca. 10,5 beträgt.

**7.** Verfahren nach Anspruch 6, wobei der pH-Wert des Elutionspuffers ca. 5,0 oder ca. 7,4 beträgt.

**8.** Verfahren nach Anspruch 1, wobei der Elutionspuffer ca. 250 mM Arginin bis ca. 1000 mM Arginin umfasst.

**9.** Verfahren nach Anspruch 8, wobei der Elutionspuffer ca. 500 mM Arginin umfasst.

**10.** Verfahren nach Anspruch 9, wobei der pH-Wert des Elutionspuffers ca. 5,0 beträgt.

**11.** Verfahren nach einem der Ansprüche 1 bis 10, ferner umfassend das Unterziehen der eluierten Serinprotease einer Reinigung mit einer Ionenaustauschsäule.

**12.** Verfahren nach einem der Ansprüche 1 bis 11, ferner umfassend das Waschen vor dem Eluieren der Serinprotease des Chromatographen mit einem Waschpuffer, der ein Salz umfasst und einen neutralen pH-Wert hat.

**13.** Kit, umfassend:

einen Sojabohnentrypsininhibitor (STI)-basierten Affinitätschromatographen, und
einen Arginin enthaltenden Elutionspuffer, der die Zwischenwirkung zwischen dem STI und der Serinprotease unterbricht.

**14.** Kit nach Anspruch 13, wobei

(i) der pH-Wert des Elutionspuffers ca. 4,5 bis ca. 10,5 beträgt; oder
(ii) der Elutionspuffer ca. 250 mM Arginin bis ca. 1000 mM Arginin umfasst.

**15.** Kit nach Anspruch 13 oder 14, ferner umfassend einen Waschpuffer, der ca. 250 mM NaCl mit einem neutralen pH-Wert umfasst.


**Revendications**

**1.** Procédé de purification d'une sérine-protéase comprenant :

le chargement de la sérine-protéase sur un chromatographe d'affinité à base d'inhibiteur de trypsine de soja (STI), et
l'élution de la sérine-protéase avec un tampon d'élution comprenant de l'arginine qui interrompt l'interaction entre le STI et la sérine-protéase.

**2.** Procédé de la revendication 1, dans lequel le tampon d'élution comprend en outre un sel, un détergent et/ou un agent chaotrope.

**3.** Procédé de la revendication 1, dans lequel la sérine-protéase est un polypeptide comprenant la séquence d'acides aminés de SEQ ID NO : 1 ou 2, ou un polypeptide ayant au moins environ 80 % d'identité de séquence avec SEQ ID NO : 1 ou 2, présentant une délétion d'au moins une partie du domaine Gla et une mutation au niveau du site actif.

**4.** Procédé de la revendication 3, dans lequel la sérine-protéase comprend la séquence d'acides aminés de SEQ ID NO : 2, ou un polypeptide ayant au moins environ 95 % d'identité de séquence avec SEQ ID NO : 2, présentant une délétion d'au moins une partie du domaine Gla et une mutation au niveau du site actif.

**5.** Procédé de la revendication 3, dans lequel la sérine-protéase comprend la séquence d'acides aminés de SEQ ID NO : 2.

**6.** Procédé de l'une quelconque des revendications 1 à 5, dans lequel le pH du tampon d'élution est d'environ 4,5 à environ 10,5.

**7.** Procédé de la revendication 6, dans lequel le pH du tampon d'élution est d'environ 5,0 ou est d'environ 7,4.

**8.** Procédé de la revendication 1, dans lequel le tampon d'élution comprend d'environ 250 mM d'arginine à environ 1000 mM d'arginine.

9. Procédé de la revendication 8, dans lequel le tampon d'élution comprend environ 500 mM d'arginine.

10. Procédé de la revendication 9, dans lequel le pH du tampon d'élution est d'environ 5,0.

11. Procédé de l'une quelconque des revendications 1 à 10, comprenant en outre la soumission de la sérine-protéase éluée à une purification par colonne échangeuse d'ions.

12. Procédé de l'une quelconque des revendications 1 à 11, comprenant en outre, avant l'élution de la sérine-protéase, le lavage du chromatographe avec un tampon de lavage qui comprend un sel et a un pH neutre.

13. Nécessaire comprenant :

un chromatographe d'affinité à base d'inhibiteur de trypsine de soja (STI), et
un tampon d'élution comprenant de l'arginine qui interrompt l'interaction entre le STI et une sérine-protéase.

14. Nécessaire de la revendication 13, dans lequel

(i) le pH du tampon d'élution est d'environ 4,5 à environ 10,5 ; ou
(ii) le tampon d'élution comprend d'environ 250 mM d'arginine à environ 1000 mM d'arginine.

15. Nécessaire de la revendication 13 ou 14, comprenant en outre un tampon de lavage comprenant 250 mM de NaCl à pH neutre.

SEQ ID NO: 1:

```
Ala Asn Ser Phe Leu Phe Trp Asn Lys Tyr Lys Asp Gly Asp Gln Cys
1                   5                   10                  15
Glu Thr Ser Pro Cys Gln Asn Gln Gly Lys Cys Lys Asp Gly Leu Gly
            20                  25                  30
Glu Tyr Thr Cys Thr Cys Leu Glu Gly Phe Glu Gly Lys Asn Cys Glu
            35                  40                  45
Leu Phe Thr Arg Lys Leu Cys Ser Leu Asp Asn Gly Asp Cys Asp Gln
        50                  55                  60
Phe Cys His Glu Glu Gln Asn Ser Val Val Cys Ser Cys Ala Arg Gly
65                  70                  75                  80
Tyr Thr Leu Ala Asp Asn Gly Lys Ala Cys Ile Pro Thr Gly Pro Tyr
                85                  90                  95
Pro Cys Gly Lys Gln Thr Leu Glu Arg Arg Lys Arg Arg Lys Arg Ile
            100                 105                 110
Val Gly Gly Gln Glu Cys Lys Asp Gly Glu Cys Pro Trp Gln Ala Leu
            115                 120                 125
Leu Ile Asn Glu Glu Asn Glu Gly Phe Cys Gly Gly Thr Ile Leu Ser
            130                 135                 140
Glu Phe Tyr Ile Leu Thr Ala Ala His Cys Leu Tyr Gln Ala Lys Arg
145                 150                 155                 160
Phe Lys Val Arg Val Gly Asp Arg Asn Thr Glu Gln Glu Glu Gly Gly
                165                 170                 175
Glu Ala Val His Glu Val Glu Val Val Ile Lys His Asn Arg Phe Thr
            180                 185                 190
Lys Glu Thr Tyr Asp Phe Asp Ile Ala Val Leu Arg Leu Lys Thr Pro
            195                 200                 205
Ile Thr Phe Arg Met Asn Val Ala Pro Ala Cys Leu Pro Glu Arg Asp
    210                 215                 220
Trp Ala Glu Ser Thr Leu Met Thr Gln Lys Thr Gly Ile Val Ser Gly
225                 230                 235                 240
Phe Gly Arg Thr His Glu Lys Gly Arg Gln Ser Thr Arg Leu Lys Met
                245                 250                 255
Leu Glu Val Pro Tyr Val Asp Arg Asn Ser Cys Lys Leu Ser Ser Ser
            260                 265                 270
Phe Ile Ile Thr Gln Asn Met Phe Cys Ala Gly Tyr Asp Thr Lys Gln
        275                 280                 285
Glu Asp Ala Cys Gln Gly Asp Ala Gly Gly Pro His Val Thr Arg Phe
    290                 295                 300
Lys Asp Thr Tyr Phe Val Thr Gly Ile Val Ser Trp Gly Glu Gly Cys
305                 310                 315                 320
Ala Arg Lys Gly Lys Tyr Gly Ile Tyr Thr Lys Val Thr Ala Phe Leu
            325                 330                 335
Lys Trp Ile Asp Arg Ser Met Lys Thr Arg Gly Leu Pro Lys Ala Lys
            340                 345                 350
Ser His Ala Pro Glu Val Ile Thr Ser Ser Pro Leu Lys
            355                 360                 365
```

# FIG. 1

SEQ ID NO: 2:

```
Ala Asn Ser Phe Leu Phe Trp Asn Lys Tyr Lys Asp Gly Asp Gln Cys
1               5               10              15
Glu Thr Ser Pro Cys Gln Asn Gln Gly Lys Cys Lys Asp Gly Leu Gly
            20              25              30
Glu Tyr Thr Cys Thr Cys Leu Glu Gly Phe Glu Gly Lys Asn Cys Glu
        35              40              45
Leu Phe Thr Arg Lys Leu Cys Ser Leu Asp Asn Gly Asp Cys Asp Gln
    50              55              60
Phe Cys His Glu Glu Gln Asn Ser Val Val Cys Ser Cys Ala Arg Gly
65              70              75              80
Tyr Thr Leu Ala Asp Asn Gly Lys Ala Cys Ile Pro Thr Gly Pro Tyr
            85              90              95
Pro Cys Gly Lys Gln Thr Leu Glu Arg Ile Val Gly Gly Gln Glu Cys
        100             105             110
Lys Asp Gly Glu Cys Pro Trp Gln Ala Leu Leu Ile Asn Glu Glu Asn
        115             120             125
Glu Gly Phe Cys Gly Gly Thr Ile Leu Ser Glu Phe Tyr Ile Leu Thr
    130             135             140
Ala Ala His Cys Leu Tyr Gln Ala Lys Arg Phe Lys Val Arg Val Gly
145             150             155             160
Asp Arg Asn Thr Glu Gln Glu Glu Gly Gly Glu Ala Val His Glu Val
            165             170             175
Glu Val Val Ile Lys His Asn Arg Phe Thr Lys Glu Thr Tyr Asp Phe
            180             185             190
Asp Ile Ala Val Leu Arg Leu Lys Thr Pro Ile Thr Phe Arg Met Asn
        195             200             205
Val Ala Pro Ala Cys Leu Pro Glu Arg Asp Trp Ala Glu Ser Thr Leu
    210             215             220
Met Thr Gln Lys Thr Gly Ile Val Ser Gly Phe Gly Arg Thr His Glu
225             230             235             240
Lys Gly Arg Gln Ser Thr Arg Leu Lys Met Leu Glu Val Pro Tyr Val
            245             250             255
Asp Arg Asn Ser Cys Lys Leu Ser Ser Ser Phe Ile Ile Thr Gln Asn
        260             265             270
Met Phe Cys Ala Gly Tyr Asp Thr Lys Gln Glu Asp Ala Cys Gln Gly
        275             280             285
Asp Ala Gly Gly Pro His Val Thr Arg Phe Lys Asp Thr Tyr Phe Val
    290             295             300
Thr Gly Ile Val Ser Trp Gly Glu Gly Cys Ala Arg Lys Gly Lys Tyr
305             310             315             320
Gly Ile Tyr Thr Lys Val Thr Ala Phe Leu Lys Trp Ile Asp Arg Ser
            325             330             335
Met Lys Thr Arg Gly Leu Pro Lys Ala Lys Ser His Ala Pro Glu Val
        340             345             350
Ile Thr Ser Ser Pro Leu Lys
        355
```

**FIG. 2**

**FIG. 3**

**FIG. 4**

FIG. 5

**FIG. 6**

**FIG. 7**

**FIG. 8**

**FIG. 9**

r-Antidote     MK     r-Antidote

**FIG. 10A**

anti-HC          anti-LC

**FIG. 10B**

**FIG. 11**

**FIG. 12**

```
┌─────────────────────────────────────┐
│          1. Clarification            │
│            (0.2 μm)                  │
└─────────────────────────────────────┘
                    │
                    ▼
┌─────────────────────────────────────┐
│          2. UF: 5K MWCO              │
│             (5-10X)                  │
└─────────────────────────────────────┘
                    │
                    ▼
┌─────────────────────────────────────┐
│                                      │
│   3. Q-Sepharose FF column (500 ml)  │
│                                      │
└─────────────────────────────────────┘
                    │
                    ▼
┌─────────────────────────────────────┐
│                                      │
│           4. UF: 5K MWCO             │
│                                      │
└─────────────────────────────────────┘
                    │
                    ▼
┌─────────────────────────────────────┐
│  5. STI-Agarose affinity column (200 ml)  │
│          benzamidine elution         │
└─────────────────────────────────────┘
                    │
                    ▼
┌─────────────────────────────────────┐
│          6. UF/DF: 5K MWCO           │
│         (final buffer 1XPBS)         │
└─────────────────────────────────────┘
```

FIG. 13

**FIG. 14**

FIG. 15

MK BLK A B C D E B E STD

unknown

HC

LC

**FIG. 16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8153390 B
- US 8268783 B
- US 766652 A
- WO 61659821 A

**Non-patent literature cited in the description**

- **SAMBROOK et al.** Molecular Cloning: A Laboratory Manual. 1989
- Current Protocols In Molecular Biology. 1987
- PCR 2: A Practical Approach. 1995
- Antibodies, A Laboratory Manual. 1988
- Using Antibodies, a Laboratory Manual. 1999
- Animal Cell Culture. 1987
- Current Protocols in Molecular Biology. 1987, 30
- **HERBERT, J.M. et al.** *J Pharmacol Exp Ther.,* 1996, vol. 276 (3), 1030-8
- **TANIUCHI, Y. et al.** *Thromb Haemost.,* 1998, vol. 79 (3), 543-8
- **ERIKSSON, B.I.** *Blood,* 2005, vol. 106 (11
- *Pipeline Insight: Antithrombotics - Reaching the Untreated Prophylaxis Market,* 2007
- **TURPIE, A.G. et al.** *J. Thromb. Haemost.,* 2005, vol. 3 (11), 2479-86
- **HYLEK EM.** *Curr Opin Invest Drugs,* 2007, vol. 8 (9), 778-783
- **AGNELLI, G. et al.** *J. Thromb. Haemost.,* 2007, vol. 5 (4), 746-53
- **BETZ et al.** Inhibition of Factor Xa by a Peptidyl-$\alpha$-ketothiazole Involves Two Steps. Evidence for a Stabilizing Conformational Change. *Biochemistry,* 1999, vol. 38, 14582-14591

SEQUENCE LISTING

<110> PORTOLA PHARMACEUTICALS, INC.

<120> METHOD FOR PURIFICATION OF RECOMBINANT FACTOR XA DERIVATIVES

<130> N404431EP

<140> 13732317.6
<141> 2013-06-12

<150> 61/659,821
<151> 2012-06-14

<160> 3

<170> PatentIn version 3.5

<210> 1
<211> 365
<212> PRT
<213> Artificial Sequence

<220>
<223> Synthetic polypeptide

<400> 1

```
Ala Asn Ser Phe Leu Phe Trp Asn Lys Tyr Lys Asp Gly Asp Gln Cys
1               5                   10                  15

Glu Thr Ser Pro Cys Gln Asn Gln Gly Lys Cys Lys Asp Gly Leu Gly
            20                  25                  30

Glu Tyr Thr Cys Thr Cys Leu Glu Gly Phe Glu Gly Lys Asn Cys Glu
        35                  40                  45

Leu Phe Thr Arg Lys Leu Cys Ser Leu Asp Asn Gly Asp Cys Asp Gln
    50                  55                  60

Phe Cys His Glu Glu Gln Asn Ser Val Val Cys Ser Cys Ala Arg Gly
65                  70                  75                  80

Tyr Thr Leu Ala Asp Asn Gly Lys Ala Cys Ile Pro Thr Gly Pro Tyr
                85                  90                  95

Pro Cys Gly Lys Gln Thr Leu Glu Arg Arg Lys Arg Arg Lys Arg Ile
            100                 105                 110

Val Gly Gly Gln Glu Cys Lys Asp Gly Glu Cys Pro Trp Gln Ala Leu
            115                 120                 125

Leu Ile Asn Glu Glu Asn Glu Gly Phe Cys Gly Gly Thr Ile Leu Ser
    130                 135                 140
```

```
Glu Phe Tyr Ile Leu Thr Ala Ala His Cys Leu Tyr Gln Ala Lys Arg
145             150             155             160


Phe Lys Val Arg Val Gly Asp Arg Asn Thr Glu Gln Glu Glu Gly Gly
                165             170             175


Glu Ala Val His Glu Val Glu Val Val Ile Lys His Asn Arg Phe Thr
            180             185             190


Lys Glu Thr Tyr Asp Phe Asp Ile Ala Val Leu Arg Leu Lys Thr Pro
            195             200             205


Ile Thr Phe Arg Met Asn Val Ala Pro Ala Cys Leu Pro Glu Arg Asp
    210             215             220


Trp Ala Glu Ser Thr Leu Met Thr Gln Lys Thr Gly Ile Val Ser Gly
225             230             235             240


Phe Gly Arg Thr His Glu Lys Gly Arg Gln Ser Thr Arg Leu Lys Met
                245             250             255


Leu Glu Val Pro Tyr Val Asp Arg Asn Ser Cys Lys Leu Ser Ser Ser
            260             265             270


Phe Ile Ile Thr Gln Asn Met Phe Cys Ala Gly Tyr Asp Thr Lys Gln
            275             280             285


Glu Asp Ala Cys Gln Gly Asp Ala Gly Gly Pro His Val Thr Arg Phe
    290             295             300


Lys Asp Thr Tyr Phe Val Thr Gly Ile Val Ser Trp Gly Glu Gly Cys
305             310             315             320


Ala Arg Lys Gly Lys Tyr Gly Ile Tyr Thr Lys Val Thr Ala Phe Leu
            325             330             335


Lys Trp Ile Asp Arg Ser Met Lys Thr Arg Gly Leu Pro Lys Ala Lys
            340             345             350


Ser His Ala Pro Glu Val Ile Thr Ser Ser Pro Leu Lys
    355             360             365
```

<210> 2
<211> 359
<212> PRT
<213> Artificial Sequence

<220>

<223> Synthetic polypeptide

<400> 2

```
Ala Asn Ser Phe Leu Phe Trp Asn Lys Tyr Lys Asp Gly Asp Gln Cys
1               5               10              15

Glu Thr Ser Pro Cys Gln Asn Gln Gly Lys Cys Lys Asp Gly Leu Gly
            20              25              30

Glu Tyr Thr Cys Thr Cys Leu Glu Gly Phe Glu Gly Lys Asn Cys Glu
        35              40              45

Leu Phe Thr Arg Lys Leu Cys Ser Leu Asp Asn Gly Asp Cys Asp Gln
    50              55              60

Phe Cys His Glu Glu Gln Asn Ser Val Val Cys Ser Cys Ala Arg Gly
65              70              75              80

Tyr Thr Leu Ala Asp Asn Gly Lys Ala Cys Ile Pro Thr Gly Pro Tyr
            85              90              95

Pro Cys Gly Lys Gln Thr Leu Glu Arg Ile Val Gly Gly Gln Glu Cys
        100             105             110

Lys Asp Gly Glu Cys Pro Trp Gln Ala Leu Leu Ile Asn Glu Glu Asn
        115             120             125

Glu Gly Phe Cys Gly Gly Thr Ile Leu Ser Glu Phe Tyr Ile Leu Thr
    130             135             140

Ala Ala His Cys Leu Tyr Gln Ala Lys Arg Phe Lys Val Arg Val Gly
145             150             155             160

Asp Arg Asn Thr Glu Gln Glu Glu Gly Gly Glu Ala Val His Glu Val
            165             170             175

Glu Val Val Ile Lys His Asn Arg Phe Thr Lys Glu Thr Tyr Asp Phe
        180             185             190

Asp Ile Ala Val Leu Arg Leu Lys Thr Pro Ile Thr Phe Arg Met Asn
        195             200             205

Val Ala Pro Ala Cys Leu Pro Glu Arg Asp Trp Ala Glu Ser Thr Leu
    210             215             220

Met Thr Gln Lys Thr Gly Ile Val Ser Gly Phe Gly Arg Thr His Glu
225             230             235             240
```

```
Lys Gly Arg Gln Ser Thr Arg Leu Lys Met Leu Glu Val Pro Tyr Val
                245             250                 255

Asp Arg Asn Ser Cys Lys Leu Ser Ser Ser Phe Ile Ile Thr Gln Asn
            260             265                 270

Met Phe Cys Ala Gly Tyr Asp Thr Lys Gln Glu Asp Ala Cys Gln Gly
        275             280             285

Asp Ala Gly Gly Pro His Val Thr Arg Phe Lys Asp Thr Tyr Phe Val
    290             295             300

Thr Gly Ile Val Ser Trp Gly Glu Gly Cys Ala Arg Lys Gly Lys Tyr
305             310             315                 320

Gly Ile Tyr Thr Lys Val Thr Ala Phe Leu Lys Trp Ile Asp Arg Ser
            325             330             335

Met Lys Thr Arg Gly Leu Pro Lys Ala Lys Ser His Ala Pro Glu Val
            340             345             350

Ile Thr Ser Ser Pro Leu Lys
            355
```

<210> 3
<211> 6
<212> PRT
<213> Artificial Sequence

<220>
<223> Synthetic peptide

<400> 3

```
Arg Lys Arg Arg Lys Arg
1               5
```